# EUROPEAN PATENT APPLICATION

(11) **EP 4 778 471 A2**
(43) Date of publication of application: **22.07.2026**
(21) Application number: 26151507.6
(22) Date of filing: 13.01.2026
(51) Int. Cl.: A61B 8/00, G16H 40/40

(54) **IDENTIFIER FOR A MEDICAL DEVICE**

(30) Priority: 15.01.2025 US 202519022077
(71) Applicant: Siemens Medical Solutions USA, Inc., Malvern, PA 19355 (US)
(72) Inventor: URBANO, Joseph A., Audubon, PA, 19403 (US); SCHWARTZ KLESSEL, Jodi, Plymouth Meeting, PA, 19462 (US); DRESCHEL, William R., Plymouth Meeting, PA, 19462 (US); WEYMER JR., Raymond F., Plymouth Meeting, PA, 19462 (US)
(74) Representative: HKW Intellectual Property PartG mbB

(57) **Abstract**

For identification for medical devices, the medical device is formed by a generic electronics module and a removable identification module. By using an identifier on a removable module, the identifier may be associated with different electronics modules to form the medical device at different times. The removable module may include electronic paper, label, and/or memory for storing and/or displaying information about the medical device. The removable module may allow for settings to be provided for any connected electronics module, avoiding reconfiguring by the user. A service organization does not need to re-ship back repaired components, instead providing a replacement electronics module to form the complete medical device with the user kept identification module.

## Description

### BACKGROUND

The present document relates to identification for medical devices. Medical devices with electronics may become inoperable or in need of repair. For example, a medical diagnostic ultrasound system may no longer scan properly or have a cracked housing. An identifier is used to identify or track the medical device for repair.

One service model relies on field service technicians going onsite to replace failed components or subsystems. An inventory of spare parts and subsystems that can be accessed globally by the service organization is needed. When it comes to low-cost, portable products, this model is relatively expensive and inefficient. Portable products can be shipped at low cost, and due to the compact nature of the components, servicing them down to the subsystem level often requires highly trained personnel working within a properly equipped facility.

Another common service model involves replacing the entire system, often on a temporary basis, until the original product can be repaired and returned. This service model suits portable products because the entire product is more easily shipped to a repair facility. One downside is that patient privacy laws require that patient data be removed from the original system prior to shipping. Another downside is that the customer may have to wait for days until their system is repaired and returned to them. In the meantime, the loaner system must be inconveniently reconfigured to connect to local networks, communicate with local systems, and operate as desired for specific users. The service organization must maintain a pool of loaner systems, and the customers must manage potential inconsistencies between their own system and the loaner (e.g., user settings, network settings, account information, etc.).

### SUMMARY

By way of introduction, the preferred embodiments described below include methods, systems, and non-transitory computer readable storage media for identification for medical devices. The medical device is formed by a generic electronics module and a removable identification module. By using an identifier on a removable module, the identifier may be associated with different electronics modules to form the medical device at different times. The removable module may include electronic paper, label, and/or memory for storing and/or displaying information about the medical device. The removable module may allow for settings to be provided for any connected electronics module, avoiding reconfiguring by the user. The service organization does not need to re-ship back repaired components, instead providing a replacement electronics module to form the complete medical device with the user kept identification module.

In a first aspect, a system is provided for identification of an ultrasound scanner. A housing of electronics of the ultrasound scanner has an exterior port. A system identification module is configured to removably mate with the port. The system identification module includes a label identifying the ultrasound scanner and a memory configured to store information for the ultrasound scanner, user information, and/or patient information.

In a second aspect, a method is provided for servicing a faulty medical device. A system identification module is separated from a housing of the faulty medical device. The system identification module is connected to a housing of a replacement medical device. The replacement medical device is configured based on data from the system identification module.

In a third aspect, a system is provided for medical device identification. A connector is configured to communicatively connect with electronics of a medical device. Electronic paper connects to the connector. The electronic paper is configured to display information about the medical device.

Any one or more of the aspects or concepts summarized above or in the Illustrative Embodiments below may be used alone or in combination. The aspects or concepts described for one Illustrative Embodiment or aspect may be used in other embodiments or aspects. The aspects or concepts described for a method or system may be used in others of a system, method, computer program, or non-transitory computer readable storage medium.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a block diagram of one embodiment of an ultrasound system with a removable system identification module;
Figure 2 is a block diagram of one embodiment of a medical device with a removable system identification module;
Figure 3 illustrates an example electronic paper removable module; and
Figure 4 is a flow chart diagram of one embodiment of a method for service of a medical device using a removable identification module.

### DETAILED DESCRIPTION OF THE DRAWINGS AND PRESENTLY PREFERRED EMBODIMENTS

A system identifier is provided in a removable module. For example, an ultrasound system includes an electronics module (EM) contained within an external housing. At least part of the housing, (i.e., a System Identification Module or SIM) is easily separable from the EM and includes a label identifying the system (e.g., serial number). The EM is anonymous and easily replaceable. The SIM also includes a memory, such as a programmable storage device (PSD) and/or another storage. The memory is accessed by the EM through either a wired or wireless connection.

The memory is programmed with system and/or user identifying information. The memory may include a system serial number or other system identification. The memory may also include user settings that tailor the system operation for a specific user or set of users. The memory may store patient health information (PHI), such as images, date of birth, etc.

This arrangement allows for a low-cost, efficient method of customer service that minimizes customer downtime and the need for skilled field service technicians. By relying on an easily removable module or panel (e.g., SIM), the information that makes the system unique is stored in the SIM and automatically transferred for use by any generic EM. The EM is replaced without need for on-site technicians or local parts depots. From the user's perspective, user presets, account login information, network settings, system configuration settings, patient information, study images, etc. are maintained locally and privately and available regardless of the EM to which the SIM is connected. The user may easily remove the SIM from one faulty EM and add it to a replacement EM.

An ultrasound scanner, imager, or system is used as an example herein. Other medical devices with electronics may be used instead, such as EKG, sensors, scanners, pressure cuffs, pulse sensors, endoscopes, probes, or catheters. The medical device is portable, such as carriable by a person, or is cart mounted. Larger medical devices may be used.

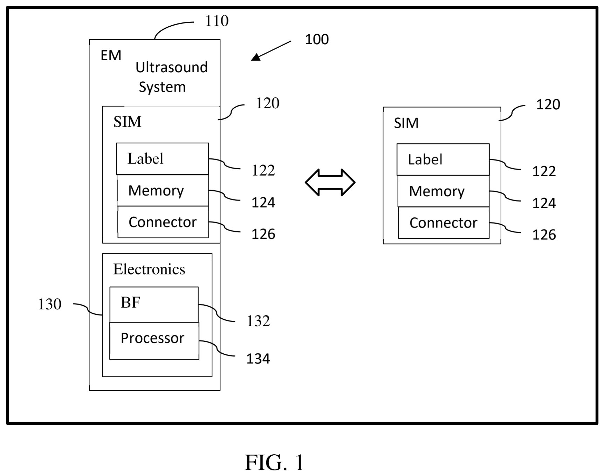

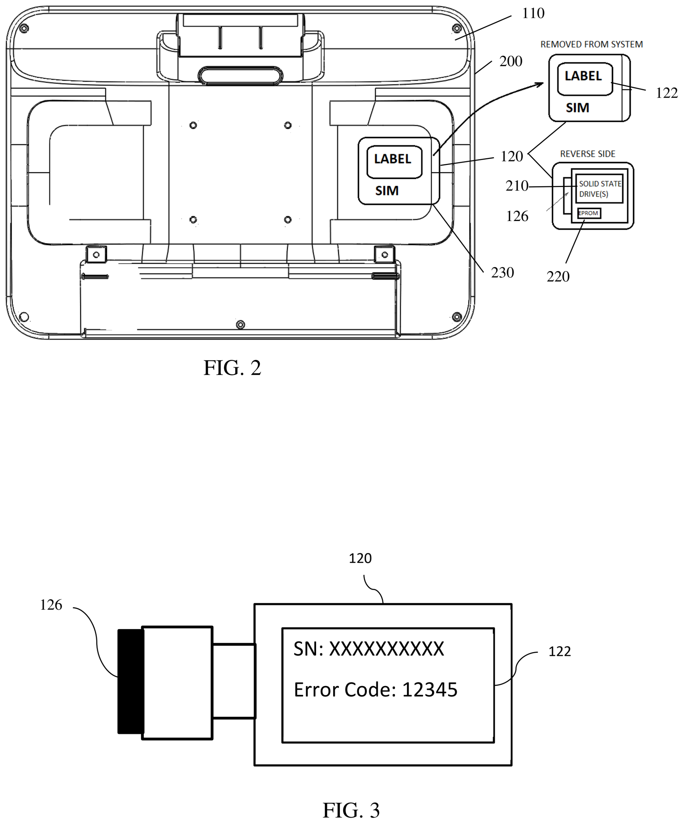

Figure 1 shows one embodiment of a system for identification of an ultrasound scanner 100. A removable module includes the identifier for the medical device (e.g., portable ultrasound scanner) and may be connected with any of various generic electronics modules to form the medical device at a given time (see Figure 2).

The ultrasound scanner 100 includes an electronics module 110 with the electronics 130 and a SIM 120. Additional, different, and/or fewer components may be provided. For example, a detachable ultrasound probe is provided.

The electronics 130 includes circuits, processor, controller, sensor, interface, communications, and/or other components for interacting with a user, sensing or scanning a patient, and/or communicating with a computer network and/or memory. In the ultrasound example, the electronics 130 includes a beamformer 132 and a processor 134. Amplifiers, filters, analog-to-digital converters, ultrasound probe, array, scan converter, and/or other electronics may be provided. Any circuitry or components for operation of the medical device are provided. Any circuitry or components for communication with the user, patient, and/or other systems (e.g., computer network, database, or medical health record) are provided. For example, a display may be provided.

The electronics 130, such as the processor 134 as a controller, electrically connects with the SIM 120. The connection may be wired. For example, a connector 126, wires, traces, buss, and/or other components provides for electronic communication to and/or from the SIM 120 with the processor 134. Any data stored in the SIM 120 may be available to the processor 134 for operation of the electronics 130. The processor 134 may be able to transfer data to the memory 126 of the SIM 120. In another example, the connector 126 is a wireless transceiver, and the electronics 130 includes another wireless transceiver. The communication between the electronics 130 and the SIM 120 is performed wirelessly. Data may be communicated to and/or from the memory 124 wirelessly. For example, the electronics 130 is configured to access the memory 120 wirelessly for any information used to configure, operate, or output by the electronics 130.

The ultrasound scanner 100 includes a housing 200 (see Figure 2). The housing 200 houses the EM 110 (e.g., the electronics 130) of the ultrasound scanner 100.

The housing 200 is plastic, fiberglass, metal, rubber, epoxy, resin, other material, or combinations thereof. A single piece or multiple pieces form the housing 200. For multiple pieces, snap fit, magnets, screws, glue, epoxy, nuts and bolts, or other now known or later developed connections between different pieces are provided.

Buttons, sliders, keyboard, knobs, touchpad, or other input devices may be included on or in the housing 200. For example, a touch sensitive display is provided. Output devices, such as a display screen, light emitting diode, or speaker may be provided.

The housing 200 is shaped and sized for carrying by the user. Figure 2 shows an example where the housing 200 is sized as a briefcase or laptop computer. A handle or handles for carrying by one or more people for portability is provided. In other implementations, the housing 200 is sized for portability on a cart, such as sized and/or shaped as a desktop computer.

In an alternative, the housing 200 is shaped and sized for being held by a hand of a user. The housing 200 is for handheld use external to the patient. The sonographer grips or holds the housing 200 for scanning a patient along the surface of the skin. In alternative embodiments, the housing 200 is a part of an endocavity, intraoperative, or other transducer housing with a portion of the housing used internally of the patient. The ergonomic aspects of the transducer housing are shaped for user gripping within the patient (e.g., intraoperative probe) or for holding a portion of the probe that is external to the patient (e.g., transesophageal probe).

The housing 200 is of any size but may be less than ten inches along the longest dimension in one implementation, less than eighteen inches along the longest dimension in another implementation, or less than twenty-four inches in yet another implementation. Larger sizes may be provided. By having a smaller size or volume, the ultrasound scanner 100 may more easily be manipulated by the sonographer to scan a patient and/or carried by a user to or from the patient.

The housing 110 includes an exterior port 230. A hole, indentation, slot, or other location for housing at least part of the SIM 120 in the housing 200 is provided. The exterior port 230 is sized to accept the entire SIM 120, such as being an indentation. The depth of the indentation may correspond to or be similar (slightly deeper) to a depth of the SIM 120 so that one surface of the SIM 120 is adjacent a surrounding surface of the housing 200 to form a continuous or smooth outer surface. In another embodiment, the SIM 120 slides into the indentation with part sticking out. Alternatively, a lid or cover with a clear or see-through window is placed over the SIM 120 while the SIM 120 is positioned in the indentation of the housing 200.

The connection is releasable. The SIM 120 releasably connects with the housing 200. A latch, snap, pressure, release, or other mechanism holds the SIM 120 to the housing 200 but allows for the SIM 120 to be removed from the housing 200 so that no physical or electrical direct connection remains (see Figures 1 and 2). In one approach, one or more screw holes are provided in the SIM 120 to align with one or more screw mounts or blocks in the housing 200. The screw(s) holds the SIM 120 in place on the housing 200. Due to the releasable connection, the SIM 120 may be separated from the housing 200 and the EM 110.

The exterior port 230 may include an electrical connector. A connector for mating with the connector 126 of the SIM is provided. For example, a receptacle for mating with the connector 126 is provided. The connector 126 allows for electrical communication between the SIM 120 and the electronics 130 (i.e., EM 110) when the SIM 120 is connected to the EM 110. The electrical connection and corresponding connector 126 are releasable, such as being a latch or plug arrangement, for disconnecting when the SIM 120 is removed from the port 230 and housing 200. In an alternative implementation, the connector 126 is a wireless transceiver, so electrical plug connection is not provided.

The SIM 120 is configured to removably mate with the port 230. By including the screw hole(s), guides, latches, holds, and/or other components, the SIM 120 is removably connected to the housing 200. The connector 126 may form part of the physical connecting, such as by plugging into a mated connector (receptacle) of the port 230, as well as electrical connection. The SIM 120 is easily separable from the EM 110, such as by unplugging, unlatching, or unscrewing.

The SIM 120 includes the label 122, memory 124, and connector 126. Additional, different, or fewer components may be provided. For example, the connector 126 is replaced by or is a wireless transceiver. As another example, the label 122 is not provided, such as where a remote display is connectable for displaying label information.

The label 122 is a display or printed information. The label 122 is positioned on the SIM 120 to be visible when mounted to or in the housing 200. The label 122 is visible to a user. In alternative embodiments, the label 122 is only fully visible when the SIM 120 is removed or partially removed from the housing 200.

The label 122 identifies the ultrasound scanner 100. For example, a serial number, model identification, electrical information (e.g., power, voltage, amperage, certification, and/or listing), tracking number, service number, contact information, and/or other information are provided. The label 122 identifies the system 100 and may give other system-related information.

In one implementation, the label 122 is a paper, plastic, or other decal material with printing (i.e., printed label). In another implementation, the label 122 is a display, such as an LCD display. Figure 3 shows an example ePaper-based label 122 on the SIM 120. The label 122 may be an intelligent or electronic paper (ePaper) display, such as an ePaper display of 1x1/2 or 2x1 inches (other sizes may be used). The ePaper display displays information even when there are no digital signals or power. The ePaper can display information unpowered so may be used in place of a decal. The ePaper may be used in combination with a printed decal.

The information displayed by an ePaper display may be changed. For example, where the SIM 120 is removed from a faulty EM 110 and connected to a replacement EM 110, the ePaper display may be updated to show information about the replacement EM 110 that is different than the faulty EM 110. The replacement EM 110 provides the new information once connected to and/or when booting with the SIM 120. This new information may be displayed with the system identification (e.g., serial number), which stays with or follows the SIM 120. Pertinent or upgraded information is displayed as part of the label 122.

In a further implementation, a QRL or another code or link is displayed. For example, a decal or ePaper display shows an optically scannable code (e.g., QRL or bar code) that links to information about the ultrasound scanner 100 or EM 110. The link may provide a system history or other relevant information. Use of ePaper may allow the code or link to be updated or changed.

In another implementation, the ePaper display as the label 122 may provide information about any fault in the EM 110. For example, a "cause of death" (e.g., error code) of the system board (e.g., EM 110 or electronics 130) is displayed where the system outputs errors or codes before becoming inoperable. Figure 3 shows an example ePaper label 122 on the SIM 120. The label 122 shows the serial number (SN) and the error code for a faulty EM).

The memory 124 is a non-transitory computer readable storage medium, such as a cache, buffer, random access memory (RAM), removable medium, hard drive, solid state memory, or another computer readable storage medium. Computer readable storage media include various types of volatile and nonvolatile storage media.

In one implementation, the memory 124 is a programmable storage. Programmable storage devices include electrically programmable read only memory (EPROM), erasable EPROM, solid-state drive (SSD), trusted platform modules (TPM), hard-drives, and/or other memories.

The memory 122 is formed from one or multiple devices. For example, a single memory formatted to be addressed in a common scheme is provided. As another example, Figure 2 shows the memory 122 as two separate devices - SSD 210 and EPROM 220. The different memories (e.g., SSD 210 and EPROM 220) may store the same or different information, such as the SSD 210 storing patient information and the EPROM 220 storing other information (e.g., information for the ultrasound scanner and/or user information).

The memory 122 stores or is programmed to store ultrasound scanner information, user information, and/or patient information. Other information may be stored. Identifying information may be stored, such as the serial number for the ultrasound scanner 100. The memory 122 may be a non-transitory storage medium storing instructions for execution by the processor 134 or another processor. The instructions may be stored in a memory of the EM 110 or electronics 130.

In one implementation, the memory 124 (e.g., EPROM 220) stores ultrasound scanner information, such as network settings for connecting to a local computer network by the ultrasound scanner (e.g., Wi-Fi name and password), system configuration settings (e.g., display brightness, image size, dynamic range, and/or other operating system settings), user information (e.g., user account (e.g., user name and password)) and/or user presets (e.g., ultrasound scan or imaging settings for the user). Any of various settings, login information, network settings, user-specific information for the ultrasound scanner 100, communications information, account information, or ultrasound scanner 100 information may be stored in the SIM (e.g., in the memory 124) for use by any connected EM 110.

In an additional or different implementation, the memory 124 (e.g., SSD 210) stores patient information. The patient information may be images (e.g., ultrasound images), patient identification, patient orders, test results, and/or other information, such as information from, in, or for a patient health record. Patient information gathered for and/or created during a previous use of the medical device (e.g., ultrasound scan) for a patient is stored.

The patient information is accessible by the EM 110 through the connector 126. For an examination of a patient, the patient information is accessed to configure the scan and/or compare results.

The patient information may be in an encrypted format. The user may provide an access code or password to unencrypt. Similarly, other information (e.g., user account and network settings) may be encrypted in the memory 124.

Since the SIM 120 is removed from a faulty EM 110, the SIM 120 may remain at a local facility while the EM 110 is shipped off site. Keeping the SIM 120 locally maintains compliance with data privacy policies and regulations. The patient information is stored on the memory 124 of the SIM 120 and not the EM 110. The part (i.e., EM 110) sent from the local facility does not have any patient information, which is stored on the removed SIM 120 staying at the local facility. The patient health information as well as account login and network access information are always kept at the user site and not stored on the EM 110 to ensure data privacy.

Figure 3 shows an example system for medical device identification. The SIM 120 is provided separately from the EM 110 but may be connected to the EM 110.

The SIM 120 includes the connector 126 and the label 122. The connector 126 is configured to communicatively connect with electronics of the medical device, such as being a plug or wired connector or being a wireless transceiver for electrical connection with the EM portion. The label 122 has identification for the medical device. In one approach, the label 122 is epaper connected to the connector 126. The epaper is configured, by a processor of the SIM 120 or the EM 110, to display information about the medical device, such as the serial number or other identifying information. An error code or other information may also or alternatively be displayed. The epaper displayed information may include the medical device identification, medical device operation parameters, a visual code for a link about the medical device, and/or an error code.

The housing 200 of the medical device is configured by shape and/or connector (e.g., receptacle) to mate with the connector 126 and epaper (i.e., mate with the SIM 120). The connector 126 and epaper are configured for releasable connection with the housing 200. The housing 200 is for any medical device.

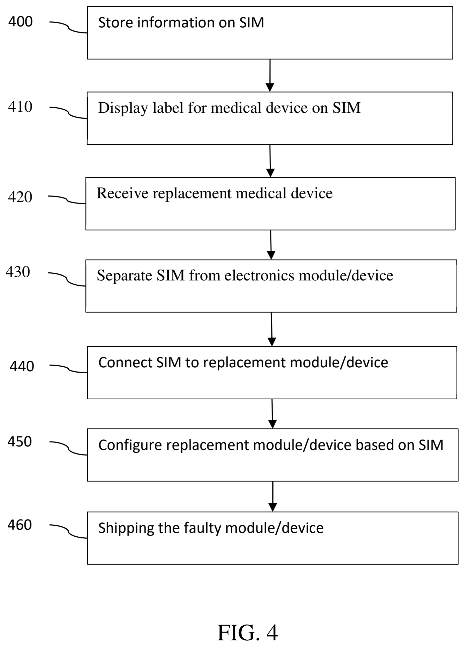

Figure 4 is a flow chart of one embodiment of a method for servicing a faulty medical device, such as a portable ultrasound system. The removable SIM enables a lower cost and more efficient service model. The SIM allows the use of a central spare parts storage facility but without costly travel and labor of a skilled field service technician. Portable products without SIMs allow a service company to avoid maintaining a loaner pool, which doubles the cost of shipping. Instead, replacement EMs are used rather than loaned EMs. The customers are not inconvenienced with loaner inconsistencies and added shipping hassles.

Additional, different, or fewer acts may be provided. For example, acts 400, 410, 420, and/or 460 are not provided. Acts from the manufacturer or service company perspective may be included, such as shipping a replacement EM or repairing a faulty EM to then later be used as a replacement EM.

The acts are performed in the order shown (top-to-bottom or numerical) or different orders. For example, act 420 may be performed after act 430. As another example, act 460 may be performed prior to acts 440 and/or 450. Act 400 may be performed before, after, or simultaneously with act 410.

The acts make use of the SIM of Figures 1-3. Other removable SIMs may be used.

In act 400, the medical device stores information on the SIM. A processor, following executed instructions, causes information to be stored on the memory of the SIM. For example, patient information, user information (e.g., account and network data), medical device information, and/or other information is stored. For patient information, other information, or all the information, the information is not stored in any memory of the faulty medical device. The memory on the EM is cleared upon storage on the SIM or when a fault is detected.

In act 410, a label on the SIM displays an identification of the medical device. The serial number, model number, and/or other product decal information is displayed. The SIM, as connected to the EM, displays the identification. The SIM and the EM together make the medical device. The SIM is the part that includes all, some, or a copy of the medical device identification. Should the EM become faulty, the label on the SIM displays the identification of the faulty medical device.

In act 420, a user receives a replacement medical device. The user reports a faulty medical device. In response, a manufacturer or service company sends a replacement EM without a SIM or with a dummy SIM. An entire system minus a SIM (i.e., the EM) may be shipped to a customer from a spare parts facility. The user receives this replacement EM.

The replacement may be received in act 420 prior to separating the SIM from the faulty EM in act 430. Alternatively, the user, in expectation of receiving the replacement EM or medical device, performs act 430 prior to receiving the replacement in act 420.

In act 430, the user or a local technician (e.g., information technology person) separates the SIM from the housing of the faulty medical device. Any screw, latch, cover, or other physical restraint is removed or undone. Any electrical connector of the SIM to the EM may be unplugged or disconnected. The physical removal may act to unplug. The user or some onsite non-technical person may remove the SIM from their system so that the SIM may be installed in the replacement EM in act 440.

In act 440, the use or technician connects the SIM to a housing of the replacement medical device (e.g., EM) received in act 420. The SIM is connected to the EM. The electrical and/or physical connector of the SIM is plugged in, and any other physical connection is made (e.g., inserting and fastening a screw). From the user's perspective, the new EM plus maintained SIM appears the same as their original medical device. The repair cost and customer downtime are both minimized.

In act 450, a processor configures the replacement medical device based on data from the SIM. For example, a processor of the electronics of the EM accesses configuration data in the memory of the SIM. This data is used to configure the medical device. The medical device with the replacement EM is configured to operate as the prior medical device (i.e., the faulty medical device prior to the fault).

The configuration may be for connection to a local network. For example, the Wi-Fi or computer network is identified in the data, and the data also includes any passwords, links, or information used to negotiate computer communications, such as with a picture archiving and communications system (PACS) or patient health information (PHI) database. As another example, the EM or replacement medical device is configured, such as setting screen brightness or other operating system settings. In another example, the EM or replacement medical device is configured with medical operation presets (e.g., scan format, depth of field, gain, and/or frequency of transmitted ultrasound) used by a user or group of users for scanning or sensing patients. In yet another example, user account access is established. The data includes user identification and password so that a user of the medical device is recognized. The data includes user information to provide access to the medical device by the user or another system associated with the user.

The configuration may provide access to patient information. Patient information may be unencrypted and displayed. The patient information from the SIM is provided on the display of the EM.

In act 460, the faulty medical device is shipped without the SIM after the separating of act 430. The faulty EM part is shipped for repair. The faulty EM is not to be fixed and sent back to the specific user, but instead to be fixed and used as a replacement for a subsequently failing EM.

The faulty EM is sent to a well-equipped repair facility where the faulty EM can be repaired by trained technicians at a manageable pace, without impacting the customer. Furthermore, since replacement EMs are generic and anonymous, like most other spare parts, companies with a variety of products, large and small, can make use of existing spare parts processes and storage facilities to manage the EM replacement. No loaner or loaner pool is necessary as the replacement is not to be returned once the faulty EM is fixed. Once the faulty EM is repaired, it is placed back in spare parts inventory, not returned to the customer.

Listed below are various Illustrative Embodiments. The Illustrative Embodiments summarize different combinations of aspects or features. Other combinations of any of the aspects or features with any other one or more of the aspects or features may be provided. Aspects or features from one type (e.g., method or system) may be used in another type (system or method).

Illustrative Embodiment 1. A system for identification of an ultrasound scanner, the system comprising: a housing of electronics of the ultrasound scanner, the housing having an exterior port; and a system identification module configured to removably mate with the port, the system identification module comprising a label identifying the ultrasound scanner and a memory configured to store information for the ultrasound scanner, user information, and/or patient information.

Illustrative Embodiment 2. The system of Illustrative Embodiment 1, wherein the housing is configured to be carried by a person.

Illustrative Embodiment 3. The system of any of Illustrative Embodiments 1-2, wherein the electronics comprises a beamformer and processor, the exterior port comprising an electrical connector communicatively connected with the processor.

Illustrative Embodiment 4. The system of any of Illustrative Embodiments 1-3, wherein the system identification module comprises a screw hole and a plug, and wherein the housing comprises a screw block and a receptacle configured to mate with the plug.

Illustrative Embodiment 5. The system of any of Illustrative Embodiments 1-4, wherein the label identifying the ultrasound scanner comprises a serial number and/or a model identification of the ultrasound scanner.

Illustrative Embodiment 6. The system of any of Illustrative Embodiments 1-5, wherein the label identifying the ultrasound scanner comprises an electronic paper display or a printed label.

Illustrative Embodiment 7. The system of any of Illustrative Embodiments 1-6, wherein the memory comprises a first memory configured to store the patient information and a second memory separate from the first memory, the second memory configured to store the information for the ultrasound scanner and the user information, the information for the ultrasound scanner comprising network settings and/or system configuration setting, and the user information comprising ultrasound scan presets and/or account information.

Illustrative Embodiment 8. The system of any of Illustrative Embodiments 1-7, wherein the memory comprises a programmable storage.

Illustrative Embodiment 9. The system of any of Illustrative Embodiments 1-8, wherein the electronics are configured to access the memory wirelessly.

Illustrative Embodiment 10. The system of any of Illustrative Embodiments 1-9, wherein the memory is configured to store the patient information in an encrypted format, the patient information comprising patient identification and/or ultrasound images.

Illustrative Embodiment 11. A method for servicing a faulty medical device, the method comprising: separating a system identification module from a housing of the faulty medical device; connecting the system identification module to a housing of a replacement medical device; and configuring the replacement medical device based on data from the system identification module.

Illustrative Embodiment 12. The method of Illustrative Embodiment 11, wherein separating comprises unplugging an electrical connector, and wherein connecting comprises plugging the electrical connector.

Illustrative Embodiment 13. The method of any of Illustrative Embodiments 11-12, wherein configuring comprises connecting to a local network, configuring the replacement medical device, setting presets of the replacement medical device, and/or establishing user account access.

Illustrative Embodiment 14. The method of any of Illustrative Embodiments 11-13, wherein configuring the replacement medical device comprises configuring to operate as the faulty medical device prior to a fault.

Illustrative Embodiment 15. The method of any of Illustrative Embodiments 11-14, further comprising receiving the replacement medical device prior to separating the system identification model, and shipping the faulty medical device without the system identification module after the separating.

Illustrative Embodiment 16. The method of any of Illustrative Embodiments 11-15, further comprising storing patient information on the system identification module and not any memory of the faulty medical device, and wherein configuring comprises providing access to the patient information.

Illustrative Embodiment 17. The method of any of Illustrative Embodiments 11-16, further comprising displaying a label on the system identification module, the label identifying the faulty medical device.

Illustrative Embodiment 18. A system for medical device identification, the system comprising: a connector configured to communicatively connect with electronics of a medical device; and electronic paper connected to the connector, the electronic paper configured to display information about the medical device.

Illustrative Embodiment 19. The system of Illustrative Embodiment 18, wherein the information comprises the medical device identification, medical device operation parameters, a visual code for a link about the medical device, and/or an error code.

Illustrative Embodiment 20. The system of any of Illustrative Embodiments 18-19, further comprising a housing of the medical device configured to mate with the connector and electronic paper, where the connector and electronic paper are configured for releasable connection with the housing.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A system for identification of an ultrasound scanner (100), the system comprising:
a housing (200) of electronics (130) of the ultrasound scanner (100), the housing (200) having an exterior port (230); and
a system identification module (120) configured to removably mate with the exterior port (230), the system identification module (120) comprising a label (122) identifying the ultrasound scanner (100) and a memory (124) configured to store information for the ultrasound scanner user information, and/or patient information.

2. The system of claim 1, wherein the housing (200) is configured to be carried by a person.

3. The system of claim 1 or 2, wherein the electronics (130) comprises a beamformer (132) and processor (134), the exterior port (230) comprising an electrical connector communicatively connected with the processor (134).

4. The system of one of claims 1 to 3, wherein the system identification module (120) comprises a screw hole and a plug, and wherein the housing (200) comprises a screw block and a receptacle configured to mate with the plug.

5. The system of one of claims 1 to 4, wherein the label (122) identifying the ultrasound scanner (100) comprises a serial number and/or a model identification of the ultrasound scanner (100), and/or
wherein the label (122) identifying the ultrasound scanner (100) comprises an electronic paper display or a printed label (122).

6. The system of one of claims 1 to 5, wherein the memory (124) comprises a first memory (210) configured to store the patient information and a second memory (220) separate from the first memory (210), the second memory (220) configured to store the information for the ultrasound scanner (100) and the user information, the information for the ultrasound scanner (100) comprising network settings and/or system configuration setting, and the user information comprising ultrasound scan presets and/or account information.

7. The system of one of claims 1 to 6, wherein the memory (124) comprises a programmable storage, and/or wherein the electronics (130) are configured to access the memory (124) wirelessly.

8. The system of one of claims 1 to 7, wherein the memory (124) is configured to store the patient information in an encrypted format, the patient information comprising patient identification and/or ultrasound images.

9. A method for servicing a faulty medical device, the method comprising:
separating (430) a system identification module (120) from a housing (200) of the faulty medical device;
connecting (440) the system identification module (120) to a housing (200) of a replacement medical device; and
configuring (450) the replacement medical device based on data from the system identification module (120).

10. The method of claim 9, wherein separating (430) comprises unplugging an electrical connector, and wherein connecting (440) comprises plugging the electrical connector.

11. The method of claim 9 or 10, wherein configuring (450) comprises connecting to a local network, configuring the replacement medical device, setting presets of the replacement medical device, and/or establishing user account access, and/or wherein configuring (450) the replacement medical device comprises configuring (450) to operate as the faulty medical device prior to a fault.

12. The method of one of claims 9 to 11, further comprising receiving (420) the replacement medical device prior to separating (430) the system identification model, and shipping (460) the faulty medical device without the system identification module (120) after the separating (430).

13. The method of one of claims 9 to 12, further comprising storing (400) patient information on the system identification module (120) and not any memory (124) of the faulty medical device, and wherein configuring (450) comprises providing access to the patient information, and/or further comprising displaying (410) a label (122) on the system identification module (120), the label (122) identifying the faulty medical device.

14. A system for medical device identification, the system comprising:
a connector (126) configured to communicatively connect with electronics (130) of a medical device; and
electronic paper (120) connected to the connector (126), the electronic paper (120) configured to display information about the medical device.

15. The system of claim 14, wherein the information comprises the medical device identification, medical device operation parameters, a visual code for a link about the medical device, and/or an error code, and/or wherein the system further comprises a housing (200) of the medical device configured to mate with the connector (126) and electronic paper (120), where the connector (126) and electronic paper (120) are configured for releasable connection with the housing (200).
